# EUROPEAN PATENT APPLICATION

(11) **EP 0 882 801 A1**
(43) Date of publication of application: **09.12.1998**
(21) Application number: 97401238.7
(22) Date of filing: 03.06.1997
(51) Int. Cl.: C12Q 1/68

(54) **Method for selecting cDNA fragments**

(71) Applicant: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventor: Zeng, Jin, F - 59000 Lille (FR); Liu, Mingfang, F - 59000 Lille (FR); Auwerx, Johan, F - 59178 Millon Fose (FR); Fruchart, Jean-Charles, F - 59130 Lambertsart (FR)
(74) Representative: Le Guen, Gérard

(57) **Abstract**

This invention consists in a method for selecting cDNA fragments generated by differential display or RNA fingerprinting comprising the steps of :
a) excising a differential band of the migration gel used for differential display or RNA fingerprinting and amplifying the corresponding cDNA fragments by PCR,
b) submitting the PCR amplified cDNA fragments to gel electrophoresis in the presence of at least one polymer-coupled DNA binding agent and/or a polypeptide capable of DNA binding and mobility retardation,
c) visualizing resolved cDNA fragments by means of a staining agent or radioactive labeling,
d) excising the desired bands separated in step b) and directly amplifying the corresponding cDNA fragments by PCR,
e) optionally purifying and analysing the amplified PCR products.

## Description

The present invention pertains to a method tor selecting cDNA fragments generated by differential display or RNA fingerprinting.

Effective methods are needed to identify and isolate genes that are differentially expressed in various cells or under altered conditions.

Differential display (Liang et al., 1992) and a closely related method, RNA fingerprinting (Welsh et al., 1992) are very useful techniques for identifying mRNA differences in cells. Although these two methods use different primer strategies, they take the same approach to isolate gene fragments from differentially expressed mRNAs by excising bands of interest from DNA sequencing gels followed by polymerase chain reaction (PCR) reamplification, Northern blot confirmation and cloning of the cDNA fragments.

A problem with these methods is that the PCR products purified from the sequencing gel are, in many cases, heterogeneous in sequence (Welsh et al., 1992). Thus, the cloned inserts, though of the expected size, are also frequently heterogeneous (Callard et al, 1994). Several approaches have been tried to circumvent this problem. One of them is the use of Northern blot for affinity capturing of cDNA (Li et al., 1994), which requires cutting blots to recover and clone the cDNA probe. However, this method needs a large amount of RNA to prepare the blots. Another approach uses plasmid dot-blotting to screen cloned cDNA fragments using cDNA PCR products as probes (Callard et al., 1994). Although the dot-blotting method can screen many cDNA clones in a short time, the results may not always be conclusive because of heterogeneity in the probe of the cDNA PCR product or because the cloned cDNA fragments were originally reverse transcription (RT)-PCR related false positives.

The selection of true positives is usually achieved by first cloning them into a PCR vector such as "TA cloning" (Invitrogen Inc.), followed by classifying clones with either restriction enzyme digestion or sequencing or single-strand conformation polymorphism (SSC) (Zhao et al., 1996). Finally, reverse Northern and Northern blot analysis are carried out to search for "true positive" clones (Mou et al, 1994). A complete post-display analysis often involves screening hundreds of individual clones. It is therefore a major bottleneck in the differential cloning methods.

The identification of true positive cDNA fragments has been recognized as the most cumbersome steps of differential display (Zhao et al. 1996). Furthermore, it is not only a complex but also an expensive and time-consuming step.

So there has been a need for a direct and simple method of classification and selection of heterogeneous cDNA fragments generated by differential display or RNA fingerprinting, wherein major distinct species of a single differential band identified by RNA fingerprinting could be directly separated and classified without cloning.

The inventors have presently solved the above-identified problem by providing an efficient method for resolving co-migrating cDNA molecules and recovering them for PCR amplification.

Thus one object of the present invention is a method for selecting cDNA fragments generated by differential display or RNA fingerprinting comprising the steps of :
a) excising a differential band of the migration gel used for differential display or RNA fingerprinting and amplifying the corresponding cDNA fragments by PCR,
b) submitting the PCR amplified cDNA fragments to gel electrophoresis in the presence of at least one polymer-coupled DNA binding agent and/or a polypeptide capable of DNA binding and mobility retardation,
c) visualizing resolved cDNA fragments by means of a staining agent or radioactive labeling,
d) excising the desired bands separated in step b) and directly amplifying the corresponding cDNA fragments by PCR,
e) optionally purifying and analysing the amplified PCR products.

The amplified PCR products may be subjected to sequencing and reverse Northern Blot, Northern Blot, ribonuclease protection assay, semi-quantitative and/or quantitative RT-PCR for verification of differential expression. In particular, the amplified PCR products may be used directly in reverse Northern Blot for rapid and high-throughput elimination of false positives and confirmation of true positives from a large number of the cDNA fragments. Finally, if desirable, the true positive cDNA fragments may also be cloned.

Another object of the present invention is a kit for selecting cDNA fragments generated by differential display or RNA fingerprinting according to the method of the invention, said kit containing :
a) a first container containing the enzymes and the reagents for PCR,
b) a second container containing at least one polymer-coupled DNA binding agent and/or one polypeptide capable of DNA binding and mobility retardation,
c) a third container containing a staining agent.

For manual collecting of desirable bands, two types of thin tubes with different diameters may be advantageously included in said kit. The tube with the larger diameter is adapted to excise desirable bands, the tube with the smaller diameter is adapted to push out the gel piece. Alternatively, for automatic collection of desirable bands, an instrument such as Rotofor system (Bio-rad) may be employed.

According to the method of the invention, the electrophoresis of step b) can be run in any kind of gel matrix, such as polysaccharide or polyacrylamide gel. It is preferably run in agarose gel.

Furthermore to achieve an optimal separation of the multiple bands, the following precautions are desirable :
- the gel may be preferably run at 10-12 V.cm⁻¹, if the electrophoresis is carried out in 0.5xTBE buffer, pH 8.0,
- slots in the gel according to step b) may be preferably 5-9 mm wide,
- a "Studier" type of apparatus may be preferably used for electrophoresis (Mc Donell et al. 1977).

The DNA binding agent used in the method of the invention may be preferably a positively charged molecule which is either an intercalating or a non-intercalating agent. It may be particularly selected from
- a planar, heterocyclic natural or synthetic molecule selected from bisbenzimide derivatives such as "Hoechst 33258", phenyl phenazinium dye, triphenylmethan dye, such as methyl green, crystal violet and malachite green;
- an antibiotic agent;
- and a DNA binding peptide.

According to the preferred embodiment of the invention, said DNA binding agent is bisbenzimide, such as Resolver Gold™ (R&D System).

Said DNA binding agent is coupled to a polymer which increases the DNA friction coefficient in electrophoresis. Said polymer may be selected from poly(C2-C4)alkylene glycol, polycaprolactone diol, poly(acrylic acid), polyacrylonitrile, poly(allylamine hydrochloride) and poly(vinyl alcohol).

In one preferred embodiment of the invention, said polymer is polyethylene glycol (PEG), polypropylene glycol, or polybutylene glycol, and is more preferably PEG having a molecular weight between 1000 and 10000, preferably between 6000 and 7500.

Many other chemical polymers may be used, which anyone skilled in the art is able to find in the Merck Index or in Aldrich or Sigma catalogues for example.

Furthermore biological molecules such as a polypeptide may be used to increase the friction of DNA in gel matrix as well.

Such a polypeptide may also contain a DNA binding site to A+T- or G+C- regions so that it has dual properties for both DNA binding and mobility retardation. In that case the presence of another DNA binding agent is not necessary.

The cDNA fragments may be visualized by means of a staining agent such as ethidium bromide, TOTO-1, YOYO-1, SYBR Green and radioactive labeling.

More than a decade ago, Müller et al., (1981) disclosed the use of dye bisbenzimide to which polyethylene glycol was bound for separating comigrating fragments.

Wawer et al., (1995) applied this method for separating two genomic DNA mutant sequences of same length. They used agarose gel electrophoresis in the presence of a DNA-binding agent bisbenzimide coupled to long chains of polyethylene glycol (PEG) 6000 for detecting sequence variation in PCR-amplified DNA molecules representing the [NiFe] hydrogenase gene. This application is also the subject-matter of the European Application EP 750 047.

In these prior art documents, the separation of the two comigrating mutant sequences was the end result of the process.

In contrast, the inventors presently developed a method which involves the recovery of desirable bands and their subsequent enrichment by direct PCR for down-stream applications, including sequencing, reverse Northern Blot and Northern Blot, as well as ribonuclease protection assay, semi-quantitative and quantitative RT-PCR for verification of differential expression.

It was known by the man skilled in the art that DNA binding agent could inhibit enzymes used in molecular biology experiments. For example, a benzopyridoindole derivative, which is an antitumor DNA binding agent, can irreversibly inhibit elongation process by different DNA polymerases including Taq DNA polymerase (Duval-Valentin et al, 1995). Another well-known DNA binding agent, isopsoralen, is generally used as a sterilizer for PCR amplification because it blocks Taq DNA polymerase (Issacs et al, 1991).

In contrast, the inventors presently discovered that surprisingly the DNA binding agent they used did not block the amplification by Taq polymerase.

So the PCR amplification can be directly carried out without prior purification.

The use of the DNA binding agent offers several advantages for screening cDNA fragments generated by differential display or RNA fingerprinting. The differential cDNA fragments are classified by standard gel electrophoresis with the dye and selected by PCR amplification rather than by cloning and doing subsequent analysis with either restriction digestion or partial sequencing or SSCP.

Firstly, the method of the invention is more cost-effective than the previous ones because it does not use expensive PCR cloning vectors and enzymes for analyzing a large number of clones.

Secondly, it is direct and rapid. One can readily process several hundreds of differential bands within a few days using the method of the invention. By contrast, methods using cloning and doing subsequent classification usually takes weeks to months.

The third and most significant benefit of the present method is that only distinct cDNA fragments are selected and further examined. In contrast, ten clones with about 70 % of redundancy have to be analyzed by the usual cloning method for each differential band in order to avoid missing an interesting one. Screening hundreds of clones is therefore a routine but cumbersome task when the previous procedures are used. Moreover, the method of the invention was found to be highly effective and reproductible.

Finally, cDNA fragments recovered from gel with the dye could be effectively amplified by PCR. The PCR products are in turn used directly in subsequent blot analysis. It was found that the cDNA fragments amplified as such were much more specific than those amplified from plasmid clones, thereby giving rise to cleaner results from down-stream analysis.

The invention is further defined in the following examples and illustrated by the annexed figures.

### LEGEND OF FIGURES

Figure 1 represents electrophoretic analysis of a differential cDNA fragment on a 1.5 % agarose gel in the absence (A) and presence (B) of Resolver Gold (R&D System). Figure 1C shows electrophoresis of three cDNA fragments re-amplified from the excised bands in (B) on a 1.5 % agarose gel without the DNA dye. Each lane contains 1-2 µg of PCR amplified cDNA fragments.

Figure 2 represents electrophoretic analysis of differential cDNA fragments on a 1.5 % agarose gel in the absence (A) and presence (B) of Resolver Gold (R&D System).

Figure 3 represents the reverse Northern Blot analysis of two differential bands (A and B) each separated into three cDNA fragments isolated from differential display gel.

### EXAMPLES

The method of the invention was applied for selecting cDNA fragments generated by differential display. These fragments correspond to genes which are differentially expressed between human visceral and subcutaneous adipose tissues.

The differential display is carried out according to Liang et al (1991) with some modifications (Linskens et al, 1995). Total RNA was isolated from human visceral and subcutaneous adipose tissues by a standard one-step method (Davis, 1986). One microgram of total RNA isolated from the tissues was used in reverse transcription reaction to synthesized first strand cDNA samples using 3' T rich primer. The cDNA samples were subjected to PCR amplification with different combinations of an anchored oligo dT primer and a random dodecamer in the presence of a radioactive labeled deoxynucleotide. The amplified PCR products from human visceral and subcutaneous fat tissues were run side-by-side on a 6% denaturing gel. The sequencing gel is dried and exposed to a X-ray film for visualizing differences.

The bands of interest which showed different intensities between human visceral and subcutaneous samples, were cut out, heated and reamplified by PCR for subsequent uses.

### Example 1:

A cDNA fragment of interest was subject to electrophoresis in the presence of the binding agent ("Resolver Gold™", R&D System) according to the manufacture's instruction. 1 unit of the agent per ml was added into 1.5 % of agarose gel precooled to 70°C or less. About 1-2 µg of DNA was loaded with bromophenol blue and electrophoresis was run in 0.5 x TBE, pH 8.0 at a voltage of 10 V.cm⁻¹. Electrophoresis was stopped as soon as the bromophenol blue migrated at a much lower speed than that at the beginning.

Afterwards, DNA was visualized by staining with 0.5 µg/ml of ethidium bromide.

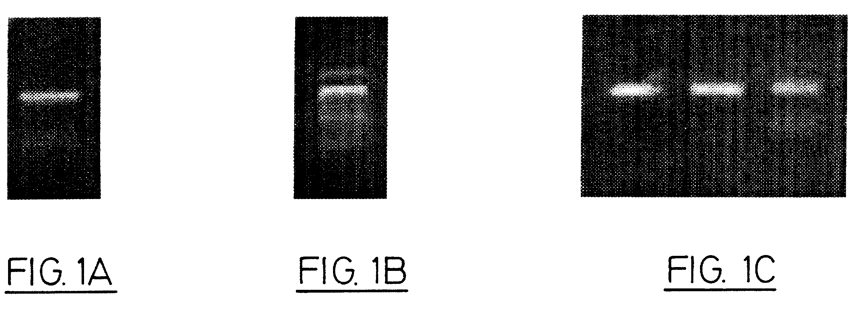

As shown in figure 1, the cDNA fragment appeared as a single band after electrophoresis in standard agarose gel (figure 1A), but was separated into three distinct bands in the presence of the DNA dye (Figure 1B).

The bands resolved by the DNA binding agent were excised from the gel. When recovering the bands, a narrow tube such as a 50 µl tip was used to stab bands of interest. Afterwards the gel slices contained in the tip were pushed into 100 µl ddH₂O by a thin tube such as a sequencing tip. The gel containing solution was then heated at 100°C for 5 min and used in PCR amplification for enrichment.

The amplified products migrated again at the same position after electrophoresis without the dye (figure 1C).

Direct sequencing on the PCR products revealed that they represented three distinct sequences.

### Example 2:

To examine its general utility, this method was tested on other candidate bands identified in the differential display project. Of fifty-two cDNA fragments isolated from differential display gels and re-amplified by PCR which represent putative human visceral- and subcutaneous- specific genes, one, eighteen, twenty-one, ten and two of them were separated into one, two, three, four and five distinct bands, respectively (table 1).

**Table 1**

| Summary of the result obtained from the separation of 52 single differential bands by Resolver Gold | |
|---|---|
| N° of resolvable bands | N° of single differential bands (%) |
| 1 | 1 (1.9) |
| 2 | 18 (34.6) |
| 3 | 21 (40.4) |
| 4 | 10 (19.2) |
| 5 | 2 (3.9) |

All the resolved bands were recovered by excising them from gels and amplified by PCR. They were used successfully in subsequent screening with blot analysis.

Thus, in this project to select regional fat-specific cDNA fragments, 64 % (33 out of 52) of them were resolved into three and more distinct bands (table 1).

This is comparable with the heterogeneity that can be obtained by cloning (Zhao et al, 1996). This significant efficiency was thought to be attributed to the fact that differential displayed cDNA fragments are derived from AT-rich 3' UTR regions of mRNA molecules to which the DNA dye preferentially binds.

### Example 3 :

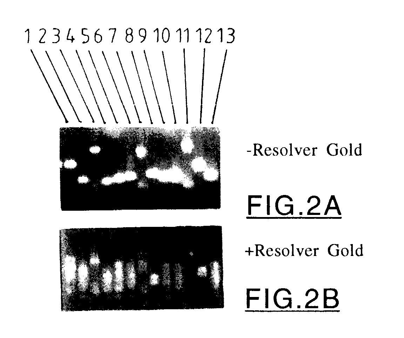

In the same project, the method of the invention was applied to the separation of thirteen differential cDNA fragments, as shown on figure 2. Electrophoresis was run in the absence (figure 2A) and in the presence (figure 2B) of Resolver Gold. The individual cDNA fragments in lanes 1, 5, 9 were separated into 5 bands, and the cDNA fragments of lane 13 were separated into 6 bands.

### Example 4 :

The PCR amplified cDNA fragments resolved by the DNA dyes according to examples 1-3 are conveniently used in a reverse Northern Blot for high-throughput screening for true positives and elimination of false positives.

A duplicate set of the cDNA fragments (each about 1-2 µg) are subjected to electrophoresis in two similar agarose gels (2 %) under standard conditions (Davis et al., 1986). Afterwards, the two gels are denatured, neutralized and blotted onto two Nylon membranes (PALL, Pall Europe Limited, England) using well known methods (Davis et al., 1986). The membranes are dried under vacuum at 80°C for two hours. Two radioactive probes are respectively made from total RNA samples isolated from human subcutaneous and visceral fat tissues essentially as described by Mou et al., 1994. Equal count of 10⁶ cpm/ml of the probes are used respectively for hybridisation of two blots under standard conditions (Davis et al., 1986). Finally, the blots are washed with 5 x SSC, 0.1 % SDS and exposed to a X-ray film. The signal intensities of the cDNA bands are then compared using a densitometer between two blots for identification of false or true positives. A signal showing a similar intensity (after correction with a known control) between two blots indicates a false positive for elimination whereas a signal displaying a significantly different intensity (after correction with a known control) indicates a potential positive for further confirmation by Northern Blot or ribonuclease protection assay or RT-PCR.

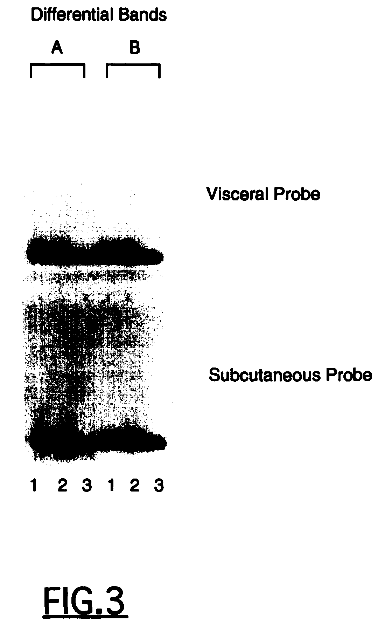

As illustrated in Figure 3, of three cDNA fragments from the differential band No A, cDNA fragments Nos 1 and 3 showed similar intensities between two blots hybridized respectively with visceral and subcutaneous probes, indicating two false positives, whereas cDNA fragment No 2 showed a higher intensity in subcutaneous blot than in visceral blot, indicating a true positive preferentially expressed in human subcutaneous fat. Likewise, of three cDNA fragments from the differential band No B, cDNA fragments Nos 1 and 3 showed similar intensities between two blots hybridized respectively with visceral and subcutaneous probes, indicating two false positives, whereas cDNa fragment No 2 showed a higher intensity in visceral blot than in subcutaneous blot, indicating a true positive that is selectively expressed in human visceral fat.

### Example 5 :

The potential positives identified in example 4 are further verified in Northern Blot analysis for differential expression. Total RNAs (10 µg) isolated from human visceral and subcutaneous fat tissues are resolved by electrophoresis in a 2 % agarose-formaldehyde gel and transferred to a nylon membrane (PALL, Pall Europe Limited, England). The filters are heated under vacuum at 80°C for two hours. The probes are prepared from the potential positive cDNA fragments using a random-primer labeling kit (Boehringer Mannheim) in the presence of α-[³²]P dCTP. Northern hybridization is carried out as described (Davis et al., 1986). Blots are washed with 5 x SSC, 0.1 % SDS and exposed to a X-ray film. A signal observed only or with a higher intensity in one of the two human adipose tissues proves the differential expression of the cDNA.

### BIBLIOGRAPHY

- Callard et al., 1994, Biotechniques, 16:1096-1102
- Davis et al., 1986, Basic methods in Molecular Biology, Elsevier Science Publishing
- Duval-Valentin et al., 1995, J. Mol. Biol., 247:847-58
- Issacs, S.T. et al., 1991, Nuc. Acids Res., 19, 109-116
- Mou. L., 1994, Biochem Phys. Res. Commun. 199, 564-9
- Li et al., 1994, Nucleic Acids Res., 22:1764-1765
- Liang et al., 1992, Science, 257:967-971
- Linskens, M.H.K, 1995, Nuc. Acids Res., 1995, 23, 3244-51
- McDonell et al., M. W. et al. ,1977, J. Mol. Biol., 110, 119-146
- Müller et al., 1981, Nucleic Acids Res., 9(1): 95-119
- Zhao et al., 1996, Biotechniques 20:400-404
- Waver et al., 1995, Nucleic Acids Res., 23(23) : 4928-4929
- Welsh et al., 1992, Nucleic Acids Res., 20:4965-4970

## Claims

1. A method for selecting cDNA fragments generated by differential display or RNA fingerprinting comprising the steps of :
a) excising a differential band of the migration gel used for differential display or RNA fingerprinting and amplifying the corresponding cDNA fragments by PCR,
b) submitting the PCR amplified cDNA fragments to gel electrophoresis in the presence of at least one polymer-coupled DNA binding agent and/or a polypeptide capable of DNA binding and mobility retardation,
c) visualizing resolved cDNA fragments by means of a staining agent or radioactive labeling,
d) excising the desired bands separated in step b) and directly amplifying the corresponding cDNA fragments by PCR,
e) optionally purifying and analysing the amplified PCR products.

2. A method according to claim 1, wherein the DNA binding agent is a positively charged molecule which is either an intercalating or a non-intercalating agent.

3. A method according to any one of claims 1 or 2, wherein the DNA binding agent is selected from
- a planar, heterocyclic natural or synthetic molecule selected from bisbenzimide derivatives, phenyl phenazinium dye, triphenylmethan dye, such as methyl green, crystal violet and malachite green;
- an antibiotic agent;
- and a DNA binding peptide.

4. A method according to any of claims 1 to 3, wherein said DNA binding agent is bisbenzimide.

5. A method according to any of claims 1 to 4, wherein said DNA binding agent is coupled to a polymer selected from poly(C2-C4)alkylene glycol, polycaprolactone diol, poly(acrylic acid), polyacrylonitrile, poly(allylamine hydrochloride), poly(vinyl alcohol) and a polypeptide.

6. A method according to claim 5, wherein said polymer is PEG having a molecular weight between 1000 and 10000, preferably between 6000 and 7500.

7. A method according to any of claims 1 to 6, wherein said PCR amplified products are analysed according to step e) by being subjected to cloning, sequencing, reverse Northern Blot, Northern Blot, ribonuclease protection assay, semi-quantitative and/or quantitative RT-PCR for verification of differential expression.

8. A method according to any of claims 1 to 7, wherein the electrophoresis of step b) is run in agarose gel.

9. A kit for selecting cDNA fragments generated by differential display or RNA fingerprinting according to the method of claims 1 to 8, said kit containing :
a) a first container containing the enzymes and the reagents for PCR,
b) a second container containing at least one polymer-coupled DNA binding agent and/or one polypeptide capable of DNA binding and mobility retardation,
c) a third container containing a staining agent.

10. A kit according to claim 9 containing, in addition to the said containers, two types of tubes with different diameters, the type of tube having a larger diameter with respect to the other tube being adapted to excise desirable bands, and the other type of tube having the smaller diameter being adapted to push out the gel piece containing said desirable bands.
